# EUROPEAN PATENT APPLICATION

(11) **EP 3 754 665 A1**
(43) Date of publication of application: **23.12.2020**
(21) Application number: 19180754.4
(22) Date of filing: 18.06.2019
(51) Int. Cl.: G16H 20/60, G16H 10/20, G16H 10/60

(54) **APPARATUS AND METHOD FOR PERSONALIZED DIET RECOMMENDATIONS**

(71) Applicant: Koninklijke Philips N.V., 5656 AG Eindhoven (NL)
(72) Inventor: TANG, Jiani (Jennie), 5656 AE Eindhoven (NL); ZHONG, Xue Feng, 5656 AE Eindhoven (NL); KUI, Xiao Yun (Shirley), 5656 AE Eindhoven (NL); MA, Henry, 5656 AE Eindhoven (NL); SUN, Wen, 5656 AE Eindhoven (NL); SU, Guangming (Roger), 5656 AE Eindhoven (NL); HIGGINS, Paul, 5656 AE Eindhoven (NL)
(74) Representative: de Haan, Poul Erik

(57) **Abstract**

A computer-implemented method for providing personalized diet recommendation to a subject comprising acquiring (202) information associated with a current dietary pattern of the subject and willingness of the subject to adjust at least one of: an aspect of their current dietary pattern and a level of physical activity of the subject, generating (204) a subject profile based on the acquired information, and providing (206) one or more diet recommendations to the subject based on the generated subject profile.

## Description

### FIELD OF THE INVENTION

The present disclosure relates to an apparatus and method for providing personalized diet recommendation to a subject.

### BACKGROUND OF THE INVENTION

A good diet is important to a person's health and well-being as it protects against malnutrition and prevents non-communicable diseases. In order to help people consume meals with balanced nutrition, there are currently many dietary guidelines available. These dietary guidelines help individuals improve and maintain their overall health and reduce the risk of chronic diseases, as well as encouraging them to shift to healthier food and beverage choices and sustain a healthy eating pattern across their lifespan. The exact make-up of a personalized balanced and healthy diet greatly varies from person to person depending on individual needs (e.g. age, gender, lifestyle, degree of physical activity, cultural context, locally available food ingredients, dietary customs as well as personal preferences). There are some applications and solutions available that have been developed to provide meal recommendations, recipes, and behavior change planning.

### SUMMARY OF THE INVENTION

As there are increasingly more dietary requirements and approaches that can be adopted by people, the factors for determining corresponding meal recommendations have become more complex. Currently available systems are able to recommend "one-size-fits-all" type recipes and behavior-altering plans, but these recommendations do not take into account a user's current dietary pattern or their willingness or readiness to make changes to their dietary pattern. Accordingly, no matter how healthy the recipe is, the user may find it difficult to implement it in their current meal pattern and therefore the relevant health goals cannot be realistically achieved. It is therefore important to provide diet recommendations to subjects by considering their willingness or readiness of effect changes, so as to ensure that their health objectives can be achieved successfully.

To better address one or more of the concerns mentioned earlier, in a first aspect, a computer-implemented method for providing personalized diet recommendation to a subject is provided. The method comprises: acquiring information associated with a current dietary pattern of the subject and willingness of the subject to adjust at least one of: an aspect of their current dietary pattern and a level of physical activity of the subject; generating a subject profile based on the acquired information, wherein the subject profile comprises at least one of: a behavior sub-profile, a meal pattern sub-profile, and a diet weak point sub-profile, and wherein the behavior sub-profile is indicative of a degree of willingness of the subject to follow a diet recommendation, the meal pattern sub-profile is indicative of an amount of required adjustment to the current meal pattern of the subject, and the diet weak point sub-profile is indicative of one or more diet weak points of the subject; and providing one or more diet recommendations to the subject based on the generated subject profile.

In some embodiments, acquiring information may comprise acquiring information from at least one of a questionnaire and an information database. In these embodiments, the information database may comprise at least one of: one or more images of food items consumed by the subject, textual information related to food items consumed by the subject, and audio information related to food items consumed by the subject.

In some embodiments, the diet weak point sub-profile may comprise at least one of: a frequency of sugary food consumption by the subject, a frequency of fried food consumption by the subject, a proportion of time using electronic devices by the subject during meal time, and an eating speed of the subject during meal time.

In some embodiments, generating the subject profile may comprise determining, based on the acquired information, a willingness and readiness value indicating whether the subject has contemplated adjusting an aspect of their current dietary pattern.

In some embodiments, determining the willingness and readiness value may be based on a level of adherence to a prescribed dietary pattern by the subject.

In some embodiments, determining a willingness and readiness value may comprise selecting from a range of values comprising: a) pre-contemplation, b) contemplation, c) preparation, d) action, and e) maintenance, wherein a) pre-contemplation indicates that the subject is not currently contemplating adjusting an aspect of their current dietary pattern, b) contemplation indicates that the subject is currently contemplating adjusting an aspect of their current dietary pattern, c) preparation indicates that the subject is currently preparing to adjust an aspect of their current dietary pattern, d) action indicates that the subject is currently performing an action to adjust an aspect of their current dietary pattern, and e) maintenance indicates that the subject is currently maintaining an action to adjust an aspect of their current dietary pattern.

In some embodiments, the method may further comprise determining whether the willingness and readiness value indicates that the subject has contemplated adjusting an aspect of their current dietary pattern. In these embodiments, if it is determined that the willingness and readiness value indicates that the subject has not contemplated adjusting an aspect of their current dietary pattern, providing one or more diet recommendations based on the generated subject profile may comprise providing educational information associated with health awareness.

In some embodiments, the method may further comprise determining whether the willingness and readiness value indicates that the subject has contemplated adjusting an aspect of their current dietary pattern. In these embodiments, if it is determined that the willingness and readiness value indicates that the subject has at least contemplated adjusting an aspect of their current dietary pattern, the method may further comprise determining, based on the diet weak point sub-profile, whether there are one or more diet weak points of the subject, and if it is determined that there are one or more diet weak points of the subject, providing one or more diet recommendations based on the generated subject profile may comprise: selecting a first diet weak point to be addressed from the one or more diet weak points; setting a desired target associated with the selected first diet weak point; and providing one or more diet recommendations based on the set desired target associated with the selected first diet weak point.

In some embodiments, providing one or more diet recommendations based on the set desired target associated with the selected first diet weak point may comprise at least one of: providing information related to one or more healthy alternatives associated with the selected first diet weak point, and providing a behavior support guide associated with the selected first diet weak point, wherein the behavior support guide comprises at least one of: one or more sub-targets associated with the selected first diet weak point, an action plan associated with the selected first diet weak point, a self-monitoring tool associated with the selected first diet weak point, a peer support tool associated with the selected first diet weak point, and a competition tool associated with the selected first diet weak point.

In some embodiments, if it is determined that there are no diet weak points of the subject, the method may further comprise: determining, based on the meal pattern sub-profile, whether an amount of required adjustment to the current meal pattern of the subject exceeds a predetermined threshold. In these embodiments, if it is determined that the amount of required adjustment to the current meal pattern of the subject does not exceed the predetermined threshold, providing one or more diet recommendations based on the generated subject profile may comprise providing one or more diet recommendations associated with optimizing a nutrient profile of the current meal pattern of the subject.

In some embodiments, the method may further comprise: determining whether the set desired target associated with the selected first diet weak point has been achieved within a predetermined time period. In these embodiments, if it is determined that the set desired target associated with the selected first diet weak point has been achieved within the predetermined time period, the method may further comprise determining whether there are one or more other diet weak points of the subject to be addressed, and if it is determined that there are no other diet weak points of the subject to be addressed, the method may further comprise determining, based on the meal pattern sub-profile, whether an amount of required adjustment to the current meal pattern of the subject exceeds a predetermined threshold, and if it is determined that the amount of required adjustment to the current meal pattern of the subject does not exceed the predetermined threshold, providing one or more diet recommendations based on the generated subject profile may comprise providing one or more diet recommendations associated with optimizing a nutrient profile of the current meal pattern of the subject.

In some embodiments, determining whether an amount of required adjustment to the current meal pattern of the subject exceeds the predetermined threshold may be based on at least one of: whether a proportion of meat-based dishes in the meal pattern exceeds a meat-based proportion threshold, whether a proportion of dishes prepared using deep-frying method or braising method exceeds a cooking method threshold, whether a taste preference of the subject is associated with sweet food items, and whether an amount of a specific type of food ingredient in the current meal pattern exceeds a food ingredient threshold.

In some embodiments, if it is determined that the amount of required adjustment of the current meal pattern of the subject exceeds the predetermined threshold, providing one or more diet recommendations based on the generated subject profile may comprise providing one or more diet recommendations associated with improving a proportion of healthy meals in the meal pattern of the subject.

In a second aspect, a computer program product comprising a computer readable medium is provided. The computer readable medium has computer readable code embodied therein, the computer readable code being configured such that, on execution by a suitable computer or processor, the computer or processor is caused to perform the method as described herein.

In a third aspect, there is provided an apparatus for providing personalized diet recommendation to a subject. The apparatus comprises a processor configured to: acquire information associated with a current dietary pattern of the subject and willingness of the subject to adjust at least one of: an aspect of their current dietary pattern and a level of physical activity of the subject; generate a subject profile based on the acquired information, wherein the subject profile comprises at least one of: a behavior sub-profile, a meal pattern sub-profile, and a diet weak point sub-profile, and wherein the behavior sub-profile is indicative of a degree of willingness of the subject to follow a diet recommendation, the meal pattern sub-profile is indicative of an amount of required adjustment to the current meal pattern of the subject, and the diet weak point sub-profile is indicative of one or more diet weak points of the subject; and provide one or more diet recommendations to the subject based on the generated subject profile.

According to the aspects and embodiments described above, the limitations of existing techniques are addressed. In particular, the above-described aspects and embodiments enable diet recommendations that take into account a subject's willingness and/or readiness to adjust an aspect of their current dietary pattern to be provided. There is thus provided an improved method and apparatus for providing diet recommendation to a subject which is based on a subject profile (including a behavior sub-profile, a meal pattern sub-profile, and a diet weak point sub-profile) so as to guide the subject to establish and sustain a balanced dietary pattern through a small-changes approach.

These and other aspects of the disclosure will be apparent from and elucidated with reference to the embodiment(s) described hereinafter.

### BRIEF DESCRIPTION OF THE DRAWINGS

For a better understanding of the embodiments, and to show more clearly how they may be carried into effect, reference will now be made, by way of example only, to the accompanying drawings, in which:
Fig. 1 is a block diagram of an apparatus for providing personalized diet recommendation to a subject according to an embodiment; and
Fig. 2 illustrates a method for providing personalized diet recommendation to a subject according to an embodiment; and
Fig. 3 is a flowchart illustrating how one or more diet recommendations can be provided to a subject, according to an embodiment.

### DETAILED DESCRIPTION OF EMBODIMENTS

As noted above, there is provided an improved apparatus and a method of operating the same which addresses the existing problems.

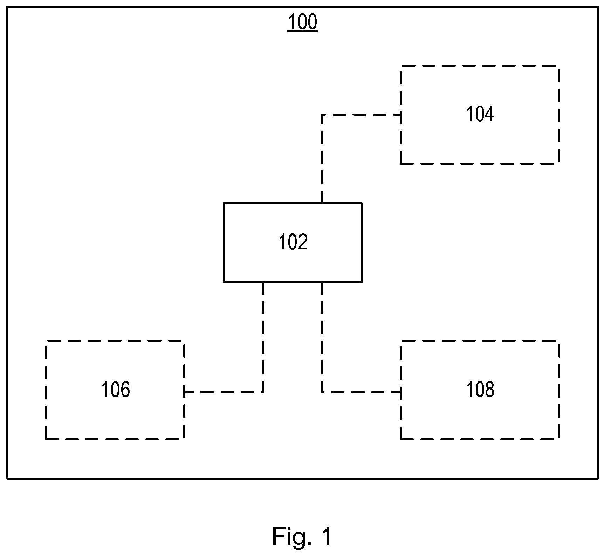

Fig. 1 shows a block diagram of an apparatus 100 according to an embodiment, which can be used for providing personalized diet recommendation to a subject. A diet recommendation may comprise at least one of: educational information associated with health awareness (e.g. a news article or a published study), a behavior support guide (e.g. an action plan, a self-monitoring tool, a peer support tool, etc.), information on food/drink alternatives, a recipe which contain instructions for the subject on how to prepare a meal, instructions on how to adjust a recipe to increase or decrease a certain type of nutrient, information and/or instruction associated with nutrient information of a meal, a dish, or a food ingredient, information and/or instruction associated with a physical activity (e.g. exercises such as running, swimming, hiking, etc.). In some embodiments, a recipe may comprise a respective amount of each of a plurality of food ingredients required (and to be consumed by the subject) and food preparation instructions, such as "oven bake 400g of potatoes for 15 minutes".

As illustrated in Fig. 1, the apparatus comprises a processor 102 that controls the operation of the apparatus 100 and that can implement the method described herein. The processor 102 can comprise one or more processors, processing units, multi-core processor or modules that are configured or programmed to control the apparatus 100 in the manner described herein. In particular implementations, the processor 102 can comprise a plurality of software and/or hardware modules that are each configured to perform, or are for performing, individual or multiple steps of the method described herein.

Briefly, the processor 102 is configured to acquire information associated with a current dietary pattern of the subject and willingness of the subject to adjust at least one of: an aspect of their current dietary pattern and a level of physical activity of the subject. Dietary pattern may comprise information associated with the quantities, proportions, variety, and/or combination of different foods and drinks in the subject's diet, as well as the frequency with which they are habitually consumed. Therefore, an aspect of a dietary pattern may be a quantity of a certain food category consumed, a frequency of a certain meal consumed, etc., for example. A dietary pattern of the subject may comprise a meal pattern of the subject as well as information related to snacks that are consumed between meals (e.g. breakfast, lunch, tea, dinner), where the meal pattern of the subject comprises information associated with the quantities, proportions, variety, frequency, and/or combination of different foods and drinks in the subject's meals. In other words, in some embodiments, the dietary pattern of the subject may comprise information associated with the quantities, proportions, variety, frequency, and/or combination of different foods and drinks in the subject's meals and also the respective information of the snacks consumed between meals.

Based on the acquired information, the processor 102 is configured to generate a subject profile and to provide one or more diet recommendations to the subject based on the generated subject profile. The subject profile comprises at least one of: a behavior sub-profile, a meal pattern sub-profile, and a diet weak point sub-profile. The behavior sub-profile is indicative of a degree of willingness and readiness of the subject to follow a diet recommendation. The meal pattern sub-profile is indicative of an amount of required adjustment to the current meal pattern of the subject. The diet weak point sub-profile is indicative of one or more diet weak points of the subject. Diet weak points relate to unhealthy dietary habits of the subject and examples of diet weak points include: "sweet food items", "fried food items", "eating too quickly during meals", "using electronic devices during meals", etc. These all relate to unhealthy behavior related to issues such as overweightness and obesity according to published studies. In this case, the one or more diet recommendations provided may be at least partly based on the diet weak points of the subject.

In some embodiments, the apparatus 100 may further comprise at least one user interface 104. Alternative or in addition, at least one user interface 104 may be external to (i.e. separate to or remote from) the apparatus 100. For example, at least one user interface 104 may be part of another device. A user interface 104 may be for use in providing a user of the apparatus 100 with information resulting from the method described herein. For example, the processor 102 may be configured to control one or more user interfaces 104 to render (or output or display) the one or more diet recommendations for the subject. Alternatively or in addition, a user interface 104 may be configured to receive a user input. For example, a user interface 104 may allow a user of the apparatus 100 to manually enter instructions, data, or information. In these embodiments, the processor 102 maybe configured to acquire the user input from one or more user interfaces 104.

A user interface 104 maybe any user interface that enables the rendering (or output or display) of information to a user of the apparatus 100. Alternatively or in addition, a user interface 104 may be any user interface that enables a user of the apparatus 100 to provide a user input, interact with and/or control the apparatus 100. For example, the user interface 104 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

In some embodiments, the apparatus 100 may comprise a memory 106. Alternatively or in addition, one or more memories 106 may be external to (i.e. separate to or remote from) the apparatus 100. For example, one or more memories 106 maybe part of another device. A memory 106 can be configured to store program code that can be executed by the processor 102 to perform the method described herein. A memory can be used to store information, data, signals and measurements acquired or made by the processor 102 of the apparatus 100. For example, a memory 106 may be used to store (for example, in a local file) information associated with the current dietary pattern of the subject. The processor 102 may be configured to control a memory 106 to store the information associated with the current dietary pattern of the subject.

In some embodiments, the apparatus 100 may comprise a communications interface (or circuitry) 108 for enabling the apparatus 100 to communicate with any interfaces, memories and/or devices that are internal or external to the apparatus 100. The communications interface 108 may communicate with any interfaces, memories and/or devices wirelessly or via a wired connection. For example, the communications interface 108 may communicate with one or more user interfaces 104 wirelessly or via a wired connection. Similarly, the communications interface 108 may communicate with the one or more memories 106 wirelessly or via a wired connection.

It will be appreciated that Fig. 1 only shows the components required to illustrate an aspect of the apparatus 100 and, in a practical implementation, the apparatus 100 may comprise alternative or additional components to those shown.

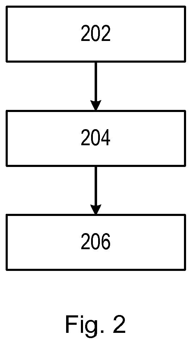

Fig. 2 illustrates a computer-implemented method for providing personalized recommendation to a subject according to an embodiment. The illustrated method can generally be performed by or under the control of processor 102 of the apparatus 100.

With reference to Fig. 2, at block 202, information associated with a current dietary pattern of the subject and willingness and readiness of the subject to adjust at least one of: an aspect of their current dietary pattern and a level of physical activity of the subject is acquired. More specifically, this information may be acquired by the processor 102 of the apparatus 100. In some embodiments, the information may be acquired from at least one of a questionnaire and an information database.

Examples of questions that may be included in the questionnaire include "How often do you eat breakfast?" and "How do you usually prepare dinner (more than 2 times a week)?". In some embodiments, the questionnaire may include one or more questions relating to the subject's attitude towards adjustment of at least an aspect of their current dietary pattern. For example, a question contained in the questionnaire may be: "Do you implement a healthy diet?" and the subject may be allowed to select one of a plurality of preset answer options including: "No, I do not intend to in the next 6 months", "No, but I intend to in the next 6 months", "No, but I intend to in the next 30 days", "Yes, I have been implementing for less than 6 months", and "Yes, I have been implementing for more than 6 months". The questionnaire may additionally or alternatively include one or more questions relating to one or more potential diet weak points (e.g. "sugary food items", "sugary drinks", "fried food items", etc.). For example, a question contained in the questionnaire may be: "How often do you consume soft drinks such as soda and energy drinks?" and the subject may be allowed to select one of a plurality of preset answer options associated with different frequencies (e.g. "once a week").

The information database may comprise at least one of: one or more images of food items consumed by the subject, textual information related to food items consumed by the subject, and audio information related to food items consumed by the subject. For example, the information database may comprise images taken by the subject of the food items or drinks he/she has consumed. As another example, the information database may comprise voice memos created by the subject related to the meals he/she has consumed.

Returning back to Fig. 2, at block 204, a subject profile is generated based on the information acquired at block 202. More specifically, the subject profile is generated by the processor 102 of the apparatus 100. The generated subject profile comprises at least one of: a behavior sub-profile, a meal pattern sub-profile, and a diet weak point sub-profile.

The behavior sub-profile is indicative of a degree of willingness and readiness of the subject follow a diet recommendation. In some embodiments, the subject profile may be generated by determining, based on the information acquired at block 202, a willingness and readiness value indicating whether the subject has contemplated adjusting an aspect of their current dietary pattern. The willingness and readiness value may be part of the behavior sub-profile of the subject. Moreover, the willingness and readiness value may be determined based on a level of adherence to a prescribed dietary pattern by the subject. For example, the level of adherence to a prescribed dietary pattern may be acquired from a mobile application of a smart phone or a tablet of the subject.

In some embodiments, determining the willingness and readiness value may comprise selecting from a range of values comprising: a) pre-contemplation, b) contemplation, c) preparation, d) action, and e) maintenance. In these embodiments, a) pre-contemplation indicates that the subject is not currently contemplating adjusting an aspect of their current dietary pattern (and has not prepared or performed an action to adjust an aspect of their current dietary pattern; b) contemplation indicates that the subject is currently contemplating adjusting an aspect of their current dietary pattern; c) preparation indicates that the subject is currently preparing to adjust an aspect of their current dietary pattern; d) action indicates that the subject is currently performing an action to adjust an aspect of their current dietary pattern; e) maintenance indicates that the subject is currently maintaining an action to adjust an aspect of their current dietary pattern. For example, as mentioned above, the questionnaire may include the question "Do you implement a healthy diet?" and the subject may be allowed to select one of a plurality of preset answer options including: "No, I do not intend to in the next 6 months", "No, but I intend to in the next 6 months", "No, but I intend to in the next 30 days", "Yes, I have been implementing for less than 6 months", and "Yes, I have been implementing for more than 6 months". In this case, the willingness and readiness value may be selected by the processor 102 as a) if the answer is "No, I do not intend to in the next 6 months", b) if the answer is "No, but I intend to in the next 6 months", c) if the answer is "No, but I intend to in the next 30 days", d) if the answer is "Yes, I have been implementing for less than 6 months", and e) if the answer is "Yes, I have been implementing for more than 6 months".

The meal pattern sub-profile is indicative of an amount of required adjustment to the current meal pattern of the subject. In some embodiments, based on the acquired information at block 202, such as the questionnaire, the processor 102 maybe configured to extract information including at least one of: a category of a dish in the meal pattern (e.g. "meaty", combi-meaty", or "veggie"), a cooking method used for preparing a dish in the meal pattern (e.g. steaming, blanching, deep-frying, braising), a taste preference of the subject (e.g. sweet or spicy), and one or more food ingredients used for preparing a dish in the meal pattern (e.g. meat and poultry, egg, vegetable, soybean products, etc.). This extracted information may be used at block 204 for generating the meal pattern sub-profile of the subject profile.

The diet weak point sub-profile is indicative of one or more diet weak points of the subject. As described with reference to Fig. 1 above, examples of diet weak point of a subject may include: "sweet food items", "fried food items", "eating too quickly during meals", "using electronic devices during meals", etc. In some embodiments, the diet weak point sub-profile may comprise at least one of: a frequency of sugary food consumption by the subject, a frequency of fried food consumption by the subject, a proportion of time using electronic devices (e.g. smartphones or tablets) during meal time, and an eating speed of the subject during meal time. As indicated above with reference to block 202, in some embodiments the questionnaire may comprise one or more questions relating to one or more potential diet weak points. For example, if the subject's answer to the question "How often do you consume soft drinks such as soda and energy drinks" is "More than three times a week", the processor 102 may determine that one of the diet weak points of the subject is "sugary drinks" and accordingly generate the subject profile such that the diet weak point sub-profile indicates "sugary drinks" as one of the diet weak points of the subject. As another example, the questionnaire may include the question "How often do you use electronic device(s) during meal times?" and if the answer provided by the subject is "More than 1.5 hours per day", the processor 102 may determine that one of the diet weak points of the subject is "using electronic devices during meals" and accordingly generate the subject profile such that the diet weak point sub-profile indicates ". In this case, the processor 102 may generate the subject profile such that the diet weak point sub-profile indicates "using electronic devices during meals" as one of the diet weak points of the subject. The determination of diet weak points of the subject may be based on one or more predetermined thresholds, such as the predetermined threshold of 1.5 hours per day of electronic device usage used in the earlier example.

Returning back to Fig. 2, at block 206, one or more diet recommendations are provided to the subject based on the subject profile generated at block 204. More specifically, one or more diet recommendations are provided by the processor 102 of the apparatus 100.

As mentioned above, at block 204 a willingness and readiness value may be determined, the willingness and readiness value being indicative of whether the subject has contemplated adjusting an aspect of their current dietary pattern. In these embodiments, the processor 102 may further determine whether the willingness and readiness value indicates that the subject has contemplated adjusting an aspect of their current dietary pattern. More specifically, in embodiments where the willingness and readiness value is selected from one of a) to e), the processor 102 of the apparatus 100 may determine, if the willingness and readiness value is one of b) to e), that the willingness readiness value indicates that the subject has contemplated adjusting an aspect of their current dietary pattern. Conversely, the processor 102 of the apparatus 100 may determine, if the willingness and readiness value is a), that the willingness and readiness value that the subject has not contemplated adjusting an aspect of their current dietary pattern.

If it is determined that the willingness and readiness value indicates that the subject has not contemplated adjusting an aspect of their current dietary pattern, providing one or more diet recommendations based on the subject profile at block 206 may comprise providing educational information associated with health awareness (e.g. articles relating to dietary health). When the subject receives such educational information associated with health awareness, they are encouraged to contemplate adjusting an aspect of their current dietary pattern.

If it is determined that the willingness and readiness value indicates that the subject has at least contemplated adjusting an aspect of their current dietary pattern, the method may further comprise determining, based on the diet weak point sub-profile, whether there are one or more diet weak points of the subject. In this case, if it is determined that there are one or more diet weak points of the subject, providing one or more diet recommendations based on the subject profile at block 206 may comprise: selecting a first diet weak point to be addressed from the one or more diet weak points; setting a desired target associated with the selected first diet weak point; and providing one or more diet recommendations based on the set desired target associated with the selected first diet weak points. The selection of a diet weak point may be performed by a user (e.g. the subject), or by the processor 102 automatically. For example, an arbitrary selection may be performed by the processor 102, or a selection based on a level of severity of each of the one or more diet weak points may be performed by the processor 102. In the example of selection based on a level of severity, standardized scales may be used for determining comparable levels of severity of each of a plurality of potential diet weak points (e.g. "sugary food items" and "fried food items" etc.).

In some embodiments, providing one or more diet recommendations based on the set desired target associated with the selected first diet weak point may comprise at least one of: providing information related to one or more healthy alternatives associated with the selected first diet weak point, and providing a behavior support guide associated with the selected first diet weak point. The behavior support guide can comprise at least one of: one or more sub-targets associated with the selected first diet weak point, an action plan associated with the selected first diet weak point, a self-monitoring tool associated with the selected first diet weak point, a peer support tool associated with the selected first diet weak point, and a competition tool associated with the selected first diet weak point.

Conversely, if it is determined that there are no diet weak points of the subject, the processor 102 of the apparatus 100 may further determine, based on the meal pattern sub-profile of the subject, whether an amount of required adjustment to the current meal pattern of the subject exceeds a predetermined threshold.

If it is determined that the amount of required adjustment to the current meal pattern of the subject does not exceed the predetermined threshold, providing one or more diet recommendations at block 206 may comprise providing one or more diet recommendations associated with optimizing a nutrient profile of the current meal pattern of the subject. For example, the one or more diet recommendations provided in this regard may include information on how high quality protein and/or specific micronutrients (e.g. calcium, iron, etc.) can be increased or maximized in the meal pattern of the subject by way of using alternative food ingredients in certain dishes or increasing/decreasing certain food ingredients in the dishes. As another example, the one or more diet recommendations provided in this regard may include information how to fine-tune energy and nutrition composition in the meals and/or dishes.

On the other hand, if it is determined that the amount of required adjustment to the current meal pattern of the subject exceeds the predetermined threshold, providing one or more diet recommendations at block 206 may comprise providing one or more diet recommendations associated with improving a proportion of healthy meals in the meal pattern of the subject. For example, the one or more diet recommendations provided in this regard may include information on how "meaty" dishes in the meal pattern can be replaced by "veggie" dishes, and/or how alternative cooking methods can be used for preparing certain dishes.

In embodiments where one of the one or more diet weak points is selected and a desired target associated with the selected diet weak point is set, the method may further comprise determining whether the set desired target associated with the selected diet weak point has been achieved within a predetermined time period. In these embodiments, if it is determine that the set desired target associated with the selected diet weak point has been achieved within the predetermined time period, the processor 102 of the apparatus 100 may be further configured to determine whether there are one or more other diet weak points of the subject to be addressed. In this scenario, other diet weak points refer to weak points that have not yet been selected or addressed, i.e. no desired target has been set for such diet weak point(s).

If it is determined that there are no other diet weak points of the subject to be addressed, the method may further comprise determining, based on the meal pattern sub-profile, whether an amount of required adjustment to the current meal pattern of the subject exceeds a predetermined threshold. In these embodiments, similar to what is described above, if it is determined that the amount of required adjustment to the current meal pattern of the subject does not exceed the predetermined threshold, providing one or more diet recommendations at block 206 may comprise providing one or more diet recommendations associated with optimizing a nutrient profile of the current meal pattern of the subject. On the other hand, if it is determined that the amount of required adjustment to the current meal pattern of the subject exceeds the predetermined threshold, providing one or more diet recommendations at block 206 may comprise providing one or more diet recommendations associated with improving a proportion of healthy meals in the meal pattern of the subject.

In some embodiments, determining whether an amount of required adjustment to the current meal pattern of the subject exceeds the predetermined threshold may be based on at least one of: whether a proportion of meat-based dishes in the meal pattern exceeds a meat-based proportion threshold, whether a proportion of dishes prepared using deep-frying method or braising method exceeds a cooking method threshold, whether a taste preference of the subject is associated with sweet food items, and whether an amount of meat-based food ingredients in the current meal pattern exceeds a food ingredient threshold. The determination may therefore be based on the meal pattern sub-profile of the subject, which may include information such as a category of a dish in the meal pattern (e.g. "meaty", combi-meaty", or "veggie"), a cooking method used for preparing a dish in the meal pattern (e.g. steaming, blanching, deep-frying, braising), a taste preference of the subject (e.g. sweet or spicy), and one or more food ingredients used for preparing a dish in the meal pattern (e.g. meat and poultry, egg, vegetable, soybean products, etc.).

Although not explicitly illustrated in Fig. 2, in some embodiments the method may comprise, subsequent to providing one or more diet recommendation to the subject based on the generated subject profile at block 206, returning to block 202 so as to acquire updated information associated with a current dietary pattern of the subject and willingness of the subject to adjust at least one of: an aspect of their current dietary pattern and a level of physical activity of the subject. In this way, a feedback loop can be formed in which a dietary pattern of the subject is continuously assessed and improved.

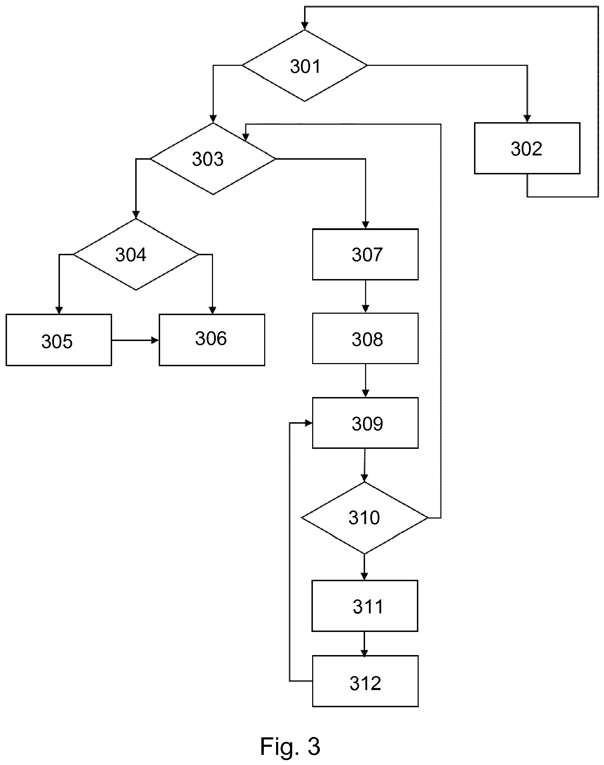

Fig. 3 is a flowchart illustrating how one or more diet recommendations can be provided to a subject, according to an embodiment. Similar to the method illustrated in Fig. 2, the method illustrated in Fig. 3 can also generally be performed by or under the control of processor 102 of the apparatus 100 of Fig. 1. The flowchart in Fig. 3 can be regarded as a detailed explanation of block 206 of Fig. 2 according to an embodiment.

In the present embodiment, the method includes determining a willingness and readiness value indicating whether the subject has contemplated adjusting an aspect of their current dietary pattern. The method begins at step 301 in which it is determined whether the determined willingness and readiness value indicates that the subject has contemplated adjusting an aspect of their current dietary pattern. As explained above with reference to Fig. 2, the willingness and readiness value may be determined by selecting from a range of values comprising: a) pre-contemplation, b) contemplation, c) preparation, d) action, and e) maintenance. In this case, at step 301 the processor 102 may determine that the willingness and readiness value indicates that the subject has at least contemplated adjusting an aspect of their current dietary pattern if the willingness and readiness value is one of b) to e), and determine that the willingness and readiness value indicates that the subject has not contemplated adjusting an aspect of their current dietary pattern if the willingness and readiness value is a).

As a result of the determination at step 301, if it is determined that the willing ness and readiness value indicates that the subject has at least contemplated adjusting an aspect of their current dietary pattern, the method process to step 302 in which one or more diet recommendations are provided by way of providing educational information associated with health awareness before returning again to step 301. On the contrary, if it is determined that the willingness and readiness value indicates that the subject has not contemplated adjusting an aspect of their current dietary pattern, the method proceeds to step 303 in which it is determined whether there are one or more unaddressed diet weak points of the subject.

As mentioned with reference to Fig. 2 above, the subject is encouraged to contemplate adjusting an aspect of their current dietary pattern when they receive the educational information at step 302. Accordingly, subsequent to step 302 when the method returns to step 301, the status of the subject may have changed such that they have now at least contemplated adjusting an aspect of their dietary pattern. Hence, the loop between step 301 and step 302 ensures that the subject is ready to receive further diet recommendation(s) by only considering their diet weak points(s) only once they have at least contemplated adjusting an aspect of their current dietary pattern.

At step 303, it is determined whether there are one or more unaddressed diet weak points of the subject. This determination may be performed by the processor 102 of the apparatus and may be based on the diet weak point sub-profile of the subject (which is a part of the generated subject profile). In the present context, an unaddressed diet weak point refers to a diet weak point for which a desired target has not been set and/or a diet weak point for which the set desired target has not been achieved. If it is determined that there are no unaddressed diet weak points of the subject, either because there are no diet weak points of the subject to begin with or because all of the diet weak points of the subject has been addressed (i.e. having a set desired target), the method proceeds to step 304 in which it is determined whether an amount of required adjustment to the current meal pattern of the subject exceeds a predetermined threshold. If it is determined that there are one or more unaddressed diet weak points of the subject, the method proceeds to step 307 at which one of the one or more unaddressed diet weak points of the subject is selected.

As mentioned above, it is determined at step 304 whether the amount of required adjustment to the current meal pattern of the subject exceeds the predetermined threshold. This determination at step 304 may be based on the meal pattern sub-profile of the subject which indicates an amount of required adjustment to the current meal pattern of the subject. In more detail, in some embodiments this determination may be based on at least one of: whether a proportion of meat-based dishes in the meal pattern of the subject exceeds a meat-based proportion threshold, whether a proportion of dishes prepared using deep-frying method or braising method exceeds a cooking method threshold, whether a taste preference of the subject is associated with sweet food items, and whether an amount of a specific type (e.g. meat-based) of food ingredient in the current meal pattern exceeds a food ingredient threshold. In other words, in some embodiment, there may be one or more criteria for determining whether the amount of required adjustment to the current meal pattern of the subject exceeds the predetermined threshold.

For example, as described with reference to Fig. 2 above, the meal pattern sub-profile of the subject may include information such as whether the dish(es) in each of the meals consumed by the subject is categorized as "meaty" (i.e. including meat as a food ingredient and the dish having a high calorie density), "combi-meaty" (i.e. including meat and vegetables as food ingredients and the dish having a medium calorie density), and "veggie" (i.e. including vegetables as a food ingredient and the dish having a low calorie density). The ranges of calorie density being classified as "high", "medium", or "low" may be predetermined in some embodiments, for example these may be set based on currently available relevant literature or studies. In this example, the proportion of meat-based dishes may be a percentage calculated by dividing the number of "meaty" and "combi-meaty" dishes in the meal pattern by the total number of total dishes in the meal pattern. The meat-based proportion threshold may be 50% and therefore in this case if the calculated proportion of meat-based dishes is equal to or more than 50%, the processor 102 may determine that the amount of adjustment to the current meal pattern of the subject exceeds the predetermined threshold. However, if the calculated proportion of meat-based dishes is less than 50%, the processor 102 may determine that the amount of adjustment to the current meal pattern of the subject does not exceed the predetermined threshold. The amount of meat-based food ingredients in the current meal pattern can be determined or calculated in similar manner, and therefore description in this regard will be omitted.

As another example, the meal pattern sub-profile may also include information associated with a cooking method used for preparing a dish in the meal pattern (e.g. steaming, blanching, deep-frying, braising). In this example, the proportion of dishes prepared using deep-frying method or braising method may be represented by a frequency of these type of dishes in the meal pattern. The deep-fry cooking method threshold may be 3 times a week and the braising cooking method threshold may be 5 times a week, and if either of these thresholds is exceeded, the processor 102 may determine that the amount of adjustment to the current meal pattern of the subject exceeds the predetermined threshold.

Alternatively or additionally, if the taste preference of the subject is associated with sweet food items, the processor 102 may determine that the amount of adjustment to the current meal pattern of the subject exceeds the predetermined threshold.

It will be appreciated that the threshold values used in the examples above (e.g. 50% as the meat-based proportion threshold) are purely exemplary and in practical implementations they may have different values. In addition, in some embodiments these threshold values may be adjusted by the processor 102 and/or a user (e.g. the subject).

If it is determined at step 304 that the amount of required adjustment to the current meal pattern of the subject exceeds the predetermined threshold, the method proceeds to step 305 at which one or more diet recommendations associated with improving a proportion of healthy meals in the meal pattern of the subject are provided. Conversely, if it is determined at step 304 that the amount of required adjustment to the current meal pattern of the subject exceeds the predetermined threshold, the method proceeds to step 306 at which one or more diet recommendations associated with optimizing a nutrient profile of the current meal pattern of the subject are provided. Moreover, subsequent to step 305, the method further proceeds to step 306.

As mentioned above, at step 307 one of the one or more unaddressed diet weak points of the subject is selected once it has been determined at step 303 that there are one or more unaddressed diet weak points of the subject. This selection may be performed by a user (e.g. the subject), or by the processor 102 automatically. Subsequent to step 307, the method proceeds to step 308 at which a desired target associated with the diet weak point selected at step 307 is set. The method then proceeds to step 309 at which one or more diet recommendations are provided based on the desired target associated with the selected diet weak point set at step 308.

As an example of steps 307 to 309, if the diet weak point selected at step 307 is "sugary drinks", a desired target associated with the selected diet weak point set at step 308 may be "Reduce sugary drinks frequency from 7 times a week to 3-4 times a week", and one of the diet recommendations provide at step 309 may be to provide information on healthy alternatives to sugary drinks (e.g. fresh juice or home-made smoothies to replace sugary drinks).

After providing one or more diet recommendations based on the set desired target associated with the selected diet weak point at step 309, the method proceeds to step 310 at which it is determined whether the desired target associated with the selected diet weak point set at step 308 has been achieved within a predetermined time period. This predetermined time period maybe, for example, a week. If it is determined at step 310 that the set desired target associated with the selected diet weak point has not been achieved within the predetermined time period, the method proceeds to step 311 at which the predetermined time period is extended (e.g. from one week to two weeks) and subsequently to step 312 at which feedback relating to a behavior learning style of the subject is received. As shown in Fig. 3, after receiving this feedback at step 312, the method returns to step 309 at which one or more diet recommendations are provided based on the set desired target. In this case, upon returning to step 309, the provision of one or more diet recommendations may be further based on the feedback received at step 312.

For example, the feedback relating to a behavior learning style of the subject received step 312 may indicate that the subject can be encouraged effectively through peer support. In this case, at step 309, based on this feedback, the one or more diet recommendations provided may include a behavior support guide related to peer support, such as a peer support tool associated with the selected diet weak point. Moreover, since the predetermined time period has been extended at step 311, once the method returns to step 310 (after performing steps 311, 312, and 309), it would be determined whether the set desired target associated with the selected diet weak point is achieved within two weeks, instead of one week.

If it is determined at step 310 that the set desired target associated with the selected diet weak point has been achieved within the predetermined time period, the method returns to step 303 at which it is determined whether there are one or more unaddressed diet weak points of the subject. As explained above, an unaddressed diet weak point refers to a diet weak point for which a desired target has not been set and/or a diet weak point for which the set desired target has not been achieved. Therefore, the one or more unaddressed diet weak points of the subject in this context do not include the diet weak point for which the set desired target has been achieved (i.e. the diet weak point that was addressed before the method return from step 310 to step 303). By establishing a loop between step 303, step 307, step 308, step 309, step 310, and step 303, the method ensures that all of the unaddressed diet weak points are addressed by way of setting desired target(s), providing one or more diet recommendations, and monitoring the progress of whether the desired target(s) have been achieved. However, it will be appreciated that in alternative embodiments the method may not necessarily return to step 303 after step 310. For example, in alternative embodiments only diet weak point(s) that have a level of severity above a predetermined threshold would be addressed (i.e. set desired target, provide diet recommendation(s), determine whether set target is achieved).

There is thus provided an improved method and apparatus for providing personalized diet recommendation to a subject, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for providing personalized diet recommendation to a subject, the method comprising:
acquiring (202) information associated with a current dietary pattern of the subject and willingness of the subject to adjust at least one of: an aspect of their current dietary pattern and a level of physical activity of the subject;
generating (204) a subject profile based on the acquired information, wherein the subject profile comprises at least one of: a behavior sub-profile, a meal pattern sub-profile, and a diet weak point sub-profile, and wherein the behavior sub-profile is indicative of a degree of willingness of the subject to follow a diet recommendation, the meal pattern sub-profile is indicative of an amount of required adjustment to the current meal pattern of the subject, and the diet weak point sub-profile is indicative of one or more diet weak points of the subject; and
providing (206) one or more diet recommendations to the subject based on the generated subject profile.

2. A computer-implemented method according to claim 1, wherein acquiring (202) information comprise acquiring information from at least one of a questionnaire and an information database,
wherein the information database comprises at least one of: one or more images of food items consumed by the subject, textual information related to food items consumed by the subject, and audio information related to food items consumed by the subject.

3. A computer-implemented method according to claim 1 or claim 2, wherein the diet weak point sub-profile comprises at least one of:
a frequency of sugary food consumption by the subject,
a frequency of fried food consumption by the subject,
a proportion of time using electronic devices by the subject during meal time, and
an eating speed of the subject during meal time.

4. A computer-implemented method according to any one of the preceding claims, wherein generating (204) the subject profile comprises determining, based on the acquired information, a willingness and readiness value indicating whether the subject has contemplated adjusting an aspect of their current dietary pattern.

5. A computer-implemented method according to claim 4, wherein determining the willingness and readiness value is based on a level of adherence to a prescribed dietary pattern by the subject.

6. A computer-implemented method according to claim 4 or claim 5, wherein determining a willingness and readiness value comprises selecting from a range of values comprising:
a) pre-contemplation,
b) contemplation,
c) preparation,
d) action, and
e) maintenance,
wherein a) pre-contemplation indicates that the subject is not currently contemplating adjusting an aspect of their current dietary pattern, b) contemplation indicates that the subject is currently contemplating adjusting an aspect of their current dietary pattern, c) preparation indicates that the subject is currently preparing to adjust an aspect of their current dietary pattern, d) action indicates that the subject is currently performing an action to adjust an aspect of their current dietary pattern, and e) maintenance indicates that the subject is currently maintaining an action to adjust an aspect of their current dietary pattern.

7. A computer-implemented method according to any one of claims 4 to 6, further comprising determining whether the willingness and readiness value indicates that the subject has contemplated adjusting an aspect of their current dietary pattern,
wherein if it is determined that the willingness and readiness value indicates that the subject has not contemplated adjusting an aspect of their current dietary pattern, providing (206) one or more diet recommendations based on the generated subject profile comprises: providing educational information associated with health awareness.

8. A computer-implemented method according to any one of claims 4 to 7, further comprising determining whether the willingness and readiness value indicates that the subject has contemplated adjusting an aspect of their current dietary pattern,
wherein if it is determined that the willingness and readiness value indicates that the subject has at least contemplated adjusting an aspect of their current dietary pattern, the method further comprises determining, based on the diet weak point sub-profile, whether there are one or more diet weak points of the subject, and
wherein if it is determined that there are one or more diet weak points of the subject, providing (206) one or more diet recommendations based on the generated subject profile comprises:
selecting a first diet weak point to be addressed from the one or more diet weak points;
setting a desired target associated with the selected first diet weak point; and
providing one or more diet recommendations based on the set desired target associated with the selected first diet weak point.

9. A computer-implemented method according to claim 8, wherein providing (206) one or more diet recommendations based on the set desired target associated with the selected first diet weak point comprises at least one of:
providing information related to one or more healthy alternatives associated with the selected first diet weak point, and
providing a behavior support guide associated with the selected first diet weak point, wherein the behavior support guide comprises at least one of: one or more sub-targets associated with the selected first diet weak point, an action plan associated with the selected first diet weak point, a self-monitoring tool associated with the selected first diet weak point, a peer support tool associated with the selected first diet weak point, and a competition tool associated with the selected first diet weak point.

10. A computer-implemented method according to claim 8 or claim 9, wherein if it is determined that there are no diet weak points of the subject, the method further comprises:
determining, based on the meal pattern sub-profile, whether an amount of required adjustment to the current meal pattern of the subject exceeds a predetermined threshold, and
wherein if it is determined that the amount of required adjustment to the current meal pattern of the subject does not exceed the predetermined threshold, providing (206) one or more diet recommendations based on the generated subject profile comprises providing one or more diet recommendations associated with optimizing a nutrient profile of the current meal pattern of the subject.

11. A computer-implemented method according to any one of claims 8 to 10, further comprising:
determining whether the set desired target associated with the selected first diet weak point has been achieved within a predetermined time period,
wherein if it is determined that the set desired target associated with the selected first diet weak point has been achieved within the predetermined time period, the method further comprises determining whether there are one or more other diet weak points of the subject to be addressed, and
wherein if it is determined that there are no other diet weak points of the subject to be addressed, the method further comprises determining, based on the meal pattern sub-profile, whether an amount of required adjustment to the current meal pattern of the subject exceeds a predetermined threshold, and
wherein if it is determined that the amount of required adjustment to the current meal pattern of the subject does not exceed the predetermined threshold, providing (206) one or more diet recommendations based on the generated subject profile comprises providing one or more diet recommendations associated with optimizing a nutrient profile of the current meal pattern of the subject.

12. A computer-implemented method according to claim 10 or claim 11, wherein determining whether an amount of required adjustment to the current meal pattern of the subject exceeds the predetermined threshold is based on at least one of: whether a proportion of meat-based dishes in the meal pattern exceeds a meat-based proportion threshold, whether a proportion of dishes prepared using deep-frying method or braising method exceeds a cooking method threshold, whether a taste preference of the subject is associated with sweet food items, and whether an amount of a specific type of food ingredient in the current meal pattern exceeds a food ingredient threshold.

13. A computer-implemented method according to any one of claims 10 to 12, wherein if it is determined that the amount of required adjustment of the current meal pattern of the subject exceeds the predetermined threshold, providing (206) one or more diet recommendations based on the generated subject profile comprises providing one or more diet recommendations associated with improving a proportion of healthy meals in the meal pattern of the subject.

14. A computer program product comprising a computer readable medium, the computer readable medium having computer readable code embodied therein, the computer readable code being configured such that, on execution by a suitable computer or processor, the computer or processor is caused to perform the method as claimed in any of the preceding claims.

15. An apparatus (100) for providing personalized diet recommendation to a subject, the apparatus comprising a processor (102) configured to:
acquire information associated with a current dietary pattern of the subject and willingness of the subject to adjust at least one of: an aspect of their current dietary pattern and a level of physical activity of the subject;
generate a subject profile based on the acquired information, wherein the subject profile comprises at least one of: a behavior sub-profile, a meal pattern sub-profile, and a diet weak point sub-profile, and wherein the behavior sub-profile is indicative of a degree of willingness of the subject to follow a diet recommendation, the meal pattern sub-profile is indicative of an amount of required adjustment to the current meal pattern of the subject, and the diet weak point sub-profile is indicative of one or more diet weak points of the subject; and
provide one or more diet recommendations to the subject based on the generated subject profile.
